# EUROPEAN PATENT APPLICATION

(11) **EP 4 516 807 A1**
(43) Date of publication of application: **05.03.2025**
(21) Application number: 23382878.9
(22) Date of filing: 28.08.2023
(51) Int. Cl.: C07K 16/28, A61K 39/395, A61P 37/06, A61K 39/00

(54) **COMPOSITIONS FOR USE IN A METHOD OF PROVIDING IMPROVED HEMATOPOIETIC STEM CELL ENGRAFTMENT**

(71) Applicant: Consorcio Centro de Investigación Biomédica en Red, 28029 Madrid (ES); Fundacion Instituto De Investigacion Sanitaria Fundacion Jimenez Diaz, 28040 Madrid (ES); Centro de Investigaciones Energéticas, Medioambientales y Tecnológicas O.A., M.P. (CIEMAT), 28040 Madrid (ES)
(72) Inventor: OJEDA PÉREZ, Isabel, 28040 Madrid (ES); SEGOVIA SANZ, José Carlos, 28040 Madrid (ES); SÁNCHEZ DOMÍNGUEZ, Rebeca, 28029 Madrid (ES); ALBERQUILLA FERNÁNDEZ, Omaira, 28040 Madrid (ES)
(74) Representative: Hoffmann Eitle

(57) **Abstract**

The present invention relates to compositions comprising at least one monoclonal antibody directed against a protein expressed on hematopoietic stem cells for use in a method of providing stem cell engraftment in a subject. The invention also relates to such compositions for use in a method of treatment of hereditary anemias, bone marrow failures, immunodeficiencies and metabolic diseases. The method comprises at least one conditioning step comprising the simultaneous or subsequent administration of the composition comprising at least one monoclonal antibody directed against a protein expressed on hematopoietic stem cells and at least one hematopoietic stem cell mobilization agent to the subject.

## Description

### TECHNICAL FIELD OF THE INVENTION

The present invention relates to a composition comprising at least one monoclonal antibody directed against a protein expressed on hematopoietic stem cells for use in a method of providing stem cell engraftment in a subject and for use in a method of treatment of hereditary anemias, bone marrow failures, immunodeficiencies and metabolic diseases in a subject. The methods comprise at least one conditioning step, wherein the conditioning step comprises the administration of the composition comprising at least one monoclonal antibody directed against a protein expressed on hematopoietic stem cells and the simultaneous or subsequent administration of at least one hematopoietic stem cell mobilization agent to the subject.

### BACKGROUND OF THE INVENTION

Hematopoietic stem cell transplantation (HSCT) is used to treat various types of cancer, blood disorders, and other serious illnesses. The goal of HSCT is to replace patient's diseased or damaged blood-forming cells with healthy stem cells that can generate new, healthy blood cells. To facilitate the engraftment of the healthy cells in the patient's body, a conditioning regimen is required. The conditioning regimen is designed to eliminate unhealthy cells, create space in the bone marrow for the transplanted stem cells to grow, and suppress the immune system to prevent rejection of the transplanted cells. Traditionally, conditioning regimens have relied on chemotherapy and/or radiation therapy to damage the DNA of diseased cells and suppress the immune system (Gyurkocza & Sandmaier, 2014). However, these treatments can cause significant side effects, such as nausea, hair loss, increased risk of infections, bleeding, organ damage, and the development of secondary cancers. Therefore, this treatment may not be suitable for all patients.

Over the past few years, there has been a trend towards using non-genotoxic conditioning regimens to provide effective conditioning while minimizing the risks associated by limiting DNA damage during the conditioning process. These treatments are particularly relevant for elder patients or those with underlying medical conditions that may make them more susceptible to the side effects of traditional conditioning regimens.

Several approaches to non-genotoxic conditioning are being explored. Those includes reduced-intensity conditioning (RIC) (lower doses of chemotherapy and/or radiation therapy), immunomodulatory conditioning, the use of monoclonal antibodies designed to recognize and bind to specific cells or proteins to selectively deplete certain immune cells or block certain signalling pathways and finally the use of drugs that induce the mobilization of HSPC. Monoclonal antibodies can be used to selectively deplete T cells or natural killer (NK) cells from the patient's immune system or block certain signalling pathways that are involved in immune activation, such as the CD40-CD40L pathway. By blocking these pathways, the patient's immune system can be suppressed without causing significant damage to their DNA. Other monoclonal antibodies that have been studied extensively include the potential utility of anti-c-kit and anti-CD47. C-kit is an integral transmembrane receptor protein expressed on the surface of hematopoietic stem cells and is involved in regulating their proliferation and survival (Chandrakasan et al., 2017). CD47, conversely, is a protein expressed on the surface of various cell types, which plays a role in immune regulation (Brunstein et al., 2011) (Majeti et al., 2009). Investigations have shown that targeted disruption of c-kit or CD47 signalling pathways may serve to the efficacy of conditioning regimens, by promoting the eradication of malignant cells and facilitating the engraftment of donor stem cells. Furthermore, evidence suggests that utilization of CD47-blocking monoclonal antibodies may serve to increase the number of regulatory T cells, which can assist in the mitigation of immune-mediated inflammatory processes following HSCT (Amsellem et al., 2003). Taken together, these findings underscore the potential of c-kit and CD47 in the optimization of non-genotoxic conditioning treatments for HSCT, representing an area of ongoing investigation and promising therapeutic development.

The use of drugs that induce the mobilization of HSPCs from the bone marrow to the peripheral blood has been proposed as a non-genotoxic conditioning strategy (Omer-Javed et al., 2022). This approach aims to create space in the bone marrow for exogenously transplanted cells by generating an empty bone marrow niche through the migration of HSPCs into the periphery. However, the percentage of engraftment obtained is limited. Treatment with hematopoietic growth factors, such as granulocyte-colony stimulating factor (G-CSF) and/or chemoquine signalling inhibitors, like plerixafor (Plx) or BIO5192, are some examples of drugs used for this purpose (Gratwohl et al., 2010).

Gene therapy is nowadays an efficient alternative for the treatment of numerous inherited hematopoietic diseases. Hematopoietic gene therapy requires the infusion of autologous cells that have been corrected ex vivo. This autologous hematopoietic stem cell transplantation (aHSCT) allows the transfer of genetically corrected HSPCs to replace diseased cells, providing a lifelong source of healthy blood cells (Anasetti et al., 2012). This autologous approach has shown effective in treating genetic disorders such as severe combined immunodeficiency (SCID), sickle cell disease (SCD), and βthalassemia(β-Tal). Autologous HSCT in gene therapy requires also hematopoietic conditioning. The application of non-genotoxic conditioning regimes will reduce dramatically the overall toxicity of the treatment and will expand its application to a broader number of hematopoietic diseases. One of the diseases that could benefit from the development of non-genotoxic conditioning treatments is erythrocyte Pyruvate Kinase Deficiency. Pyruvate kinase deficiency (PKD) is an autosomal recessive disorder, caused by genetic mutations in PKLR gene, that impair the function of pyruvate kinase, an enzyme involved in the final step of glycolysis. This impairment leads to a decreased ability of red blood cells to generate ATP, resulting in hemolytic anemia (Van Wijk & Van Solinge, 2005) (Zanella et al., 2005) (Grace et al., 2018). PKD can present with a wide range of clinical symptoms, including fatigue, jaundice, and splenomegaly. The severity of the disease can vary widely, with some individuals being asymptomatic while others require regular blood transfusions or splenectomy. The treatment options for pyruvate kinase deficiency (PKD) are mostly palliative and include blood transfusions, folic acid supplementation, and in severe cases, splenectomy (Zanella & Bianchi, 2000). The only definitive treatment for PKD is allogeneic bone marrow transplantation. An allosteric activator of the pyruvate kinase, PyruK-2, has shown promising results in non-transfusion-dependent patients but still requires further study (Enegela & Anjum, 2022). Additionally, gene therapy using lentiviral vectors is being tested in clinical trials for the treatment of PKD, with very promising results. Non-genotoxic conditioning will definitively increase the effectiveness of this treatment.

Methods of stem cell transplantation that facilitate engraftment without requiring chemo- or radiotherapy and instead applying a conditioning step comprising antibodies targeting c-kit and CD47 or other bispecific antibodies have been disclosed in WO2018140940 A1 and WO2021007266.

On the other hand, the use of drugs that induce the mobilization of HSPCs from the bone marrow niches to the periphery has been also suggested as a potential strategy to facilitate exogenous engraftment of corrected cells. For example, US2021/0100848 discloses method for mobilizing HSCs or HPCs in a subject with compounds such as granulocyte-colony stimulating factor (G-CSF), sildenafil citrate or AMD3100 (also known as (Plerixafor). However, the percentage of engraftment obtained through such mobilization step alone is limited.

Another strategy, the use of Antibody drug conjugates (ADC) carrying cell toxic drugs has also been tested as for example described in US2022/0118022. However, increased toxicity is also observed. Clinical trials are ongoing with some of these strategies.

One of the major drawbacks in the treatment of inherited hematopoietic diseases with transplantation of autologous genetically corrected cells, or with allogeneic healthy cells, is the requirement of hematopoietic conditioning of the patient to avoid endogenous competition and to generate available bone marrow niches to allow engraftment and reconstitution from the exogenous corrected (or healthy) cells. This hematopoietic conditioning is performed using chemotherapy with genotoxic agents, usually a tightly controlled Busulfan dosage. This conditioning regime is very toxic with important side effects as described above. To allow the use of gene therapy as the first treatment option for anaemias, and many other hematologic diseases, new less harmful conditioning regimens are required.

Although promising clinical trials are ongoing with some of these strategies, further research is needed to fully understand the risks and benefits of these treatments, to increase their efficacy, and to optimize their use for each individual patient.

Therefore, a high need exists for finding alternative and effective non-genotoxic conditioning regimen which avoid side effects through specificity for the hematopoietic stem and progenitors' cells and not for other cell types or tissues and improve engraftment efficiency and ultimately survival.

### SUMMARY OF THE INVENTION

The present invention therefore in one aspect relates to a composition comprising at least one monoclonal antibody directed against a protein expressed on hematopoietic cells for use in a method of providing stem cell engraftment in a subject, the method comprising at least one conditioning step that comprises simultaneously or subsequently administering to said subject
(i) the composition comprising at least one monoclonal antibody directed against a protein expressed on hematopoietic stem cells, and
(ii) at least one hematopoietic stem cell mobilization agent.

The present invention in a further aspect relates to a composition comprising at least one monoclonal antibody directed against a protein expressed on hematopoietic stem cells for use in a method of treatment of hereditary anemias, bone marrow failures, immunodeficiencies and metabolic diseases in a subject in need thereof, wherein the method of treatment comprises at least one conditioning step, wherein the conditioning step comprises the administration of the composition comprising at least one monoclonal antibody directed against a protein expressed on hematopoietic stem cells and the simultaneous or subsequent administration of at least one hematopoietic stem cell mobilization agent to the subject.

In a further aspect the present invention relates to a composition comprising at least one monoclonal antibody directed against a protein expressed on hematopoietic stem cells for use in a method of treatment of a disease that is curable by autologous or allogeneic hematopoietic progenitors stem cell transplantation, wherein the method of treatment comprises at least one conditioning step, wherein the conditioning step comprises the administration of the composition comprising at least one monoclonal antibody directed against a protein expressed on hematopoietic stem cells and the simultaneous or subsequent administration of at least one hematopoietic stem cell mobilization agent to the subject.

In one embodiment of the above aspects the composition comprising at least one monoclonal antibody directed against a protein expressed on hematopoietic stem cells and the hematopoietic stem cell mobilization agent is administered to the subject prior to the subject receiving an allogeneic or autologous hematopoietic stem cell transplant.

In one embodiment the composition comprising at least one monoclonal antibody directed against a protein expressed on hematopoietic cells and the hematopoietic stem cell mobilization agent is administered in a single dose prior to transplant.

In one embodiment of the aspects of present invention the at least one monoclonal antibody directed against a protein expressed on hematopoietic cells is selected from an anti CD47, an anti c-kit (anti CD117), an anti-CD45, an anti-CD135, an anti-CD34, an anti CD4, an anti CD3 and an anti CD38 antibody, or combinations thereof.

In one embodiment of the aspects of present invention the at least one monoclonal antibody is directed against CD47 and/or c-kit (CD117).

In one embodiment of present invention the hematopoietic stem cell mobilization agent is selected from the group consisting of Plerixafor, G-CSF, GM-CSF, GM-CSF, BIO5192, or combinations thereof.

In a preferred embodiment the hematopoietic stem cell mobilization agent is Plerixafor.

In one embodiment of present invention the administration of the at least one monoclonal antibody directed against a protein expressed on hematopoietic cells and the at least one hematopoietic stem cell mobilization agent is done subsequently, preferably with about 5 min to 3h, from about 20 min to 2h, from about 40 min to 1.5h, from about 50 min to 1h between first and second administration.

In one embodiment of present invention the administration of the composition comprising at least one monoclonal antibody directed against a protein expressed on hematopoietic cells and the at least one hematopoietic stem cell mobilization agent is done substantially simultaneously.

In one embodiment of present invention the conditioning step further comprises administering at least one agent that induces transient immunosuppression in said subject.

In one embodiment said agent that induces transient immunosuppression in said subject is selected from the group consisting of agents that inhibit CD4, CD8, CD122 and CD40L, or combinations thereof.

In one embodiment said agents that inhibit CD4, CD8, CD122 and CD40L are selected from an anti CD4 antibody, an anti CD8 antibody, an anti CD122 antibody, an anti CD40L antibody, mycophenolic acid, cyclosporine A, rapamycin, FK506 and corticosteroids, or combinations thereof.

In one embodiment of present invention the hereditary anemias are selected from B-thalassemia, sickle cell disease (SCD) or pyruvate kinase deficiency (PKD).

In a preferred embodiment the hereditary anemia is pyruvate kinase deficiency.

In one embodiment the bone marrow failures are selected from Fanconi anemia (FA), Blackfan-Diamond anemia (BDA), dyskeratosis congenita (DC), Shwachman-Diamond syndrome (SDS), congenital amegakaryocytic thrombocytopenia (CAMT) or reticular dysgenesis (RD).

In one embodiment the immunodeficiencies are selected from adenosine deaminase (ADA) deficiency, X-linked severe combined immunodeficiency (SCID), Wiskott Aldrich or X-linked chronic granulomatous disease (X-CGD).

In one embodiment the metabolic diseases are selected from metachromatic leukodystrophy or Mucopolysaccharidosis 1 or 3.

### BRIEF DESCRITION OF THE FIGURES

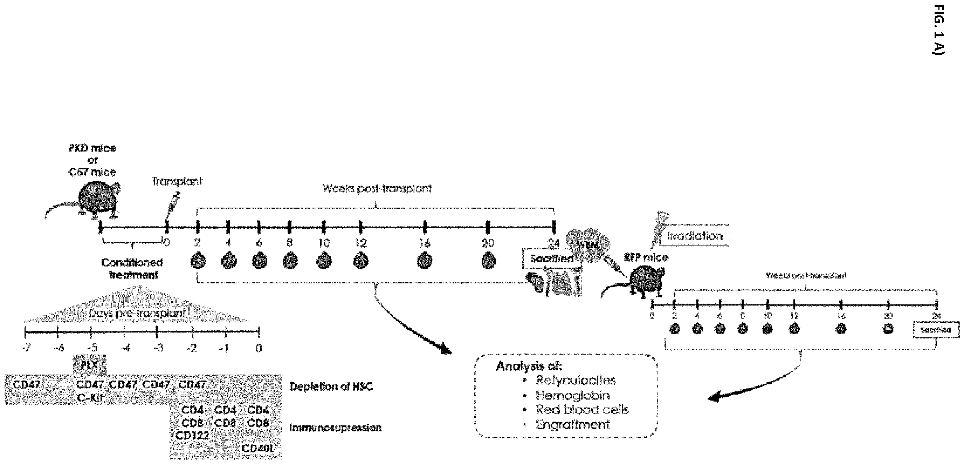

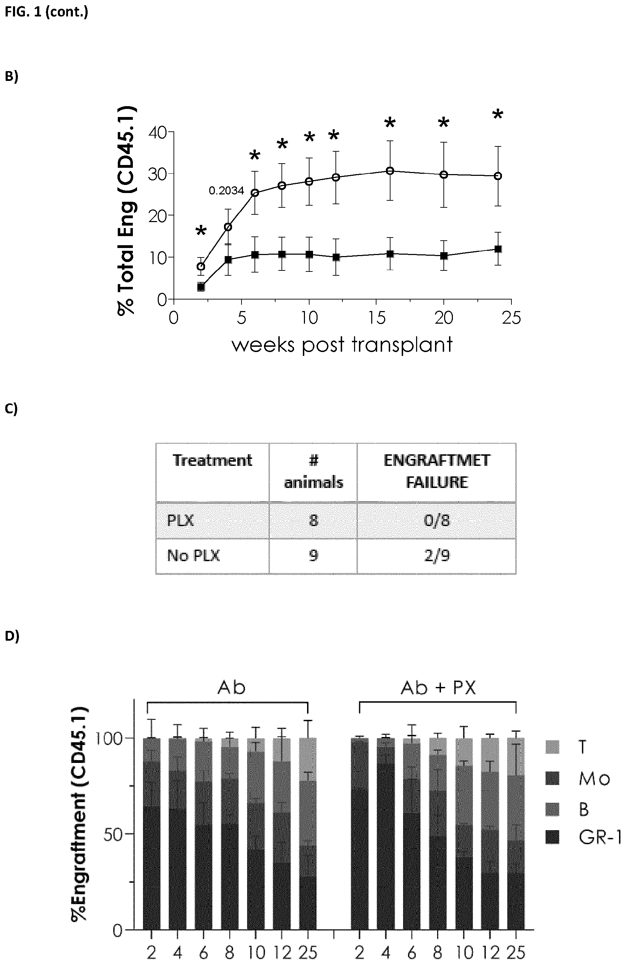

**Figure 1****: Combination of Plerixafor with Monoclonal Antibody Cocktail conditioning improves Long-Term Multi-lineage Hematopoietic Reconstitution.** A) Administration schedule for 6Ab conditioning with or without Plerixafor (PLX) regimen and experimental design. B) Peripheral blood chimerism in wild type CD45.2 transplanted with 15,000 CD45.1 LSK cells after MoAb conditioning with or without Plx. C) Engraftment failure in the experimental groups. D) Kinetics of CD45.1 lineage recovery over time on primary recipients. *p<0.05.

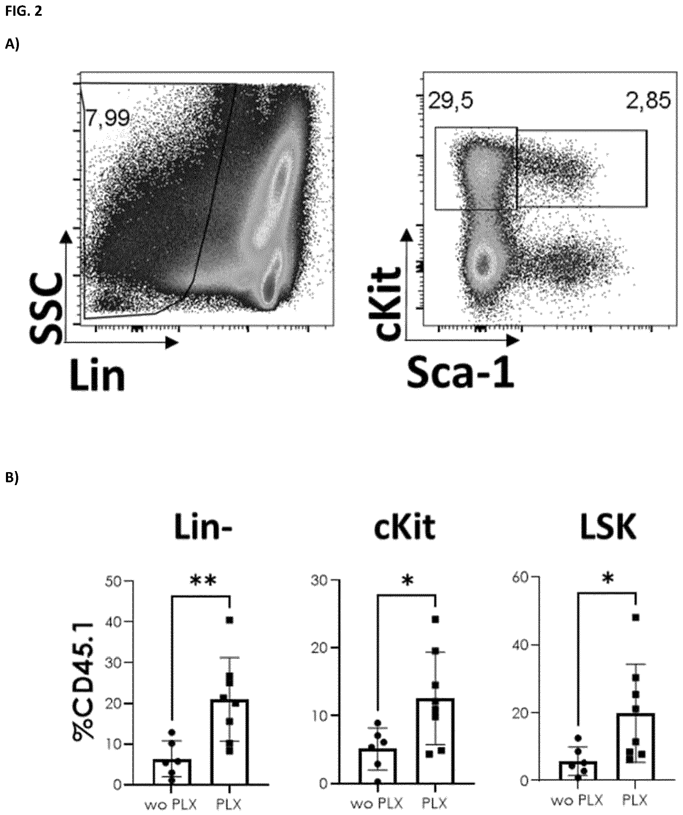

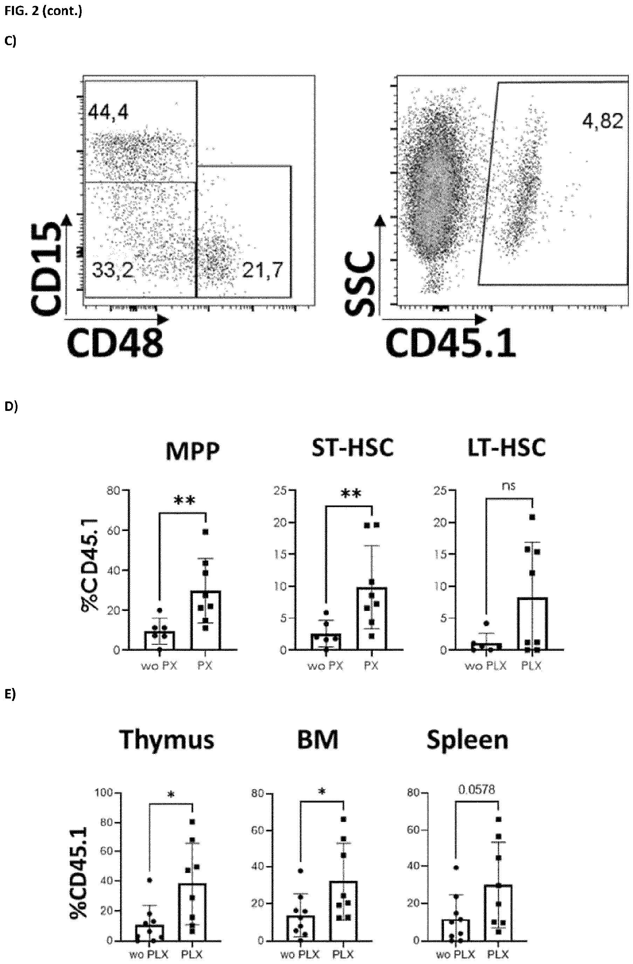

**Figure 2****: Hematopoietic engraftment in MoAb conditioned animals with and without Plerixafor 6 months post-transplant.** A) Representative dot plot showing gating strategy for the analysis of LSK and c-Kit progenitors. B) Chimerism of CD45.1 cells after 6 months within Lin-, c-kit and LSK progenitor populations. C) Representative dot plot showing SLAM based gating strategy of immature populations (MPP, ST-HSC and LT-HSC, left) and CD45.1 gating example (right); D) Percentage of CD45.1 cells after 6 months within MPP, ST-HSC and LT-HSC immature progenitors. E) Hematopoietic chimerism 6 months post-transplant in whole BM, spleen, and thymus. ns, non-significative, *p<0.05, **p<0.01.

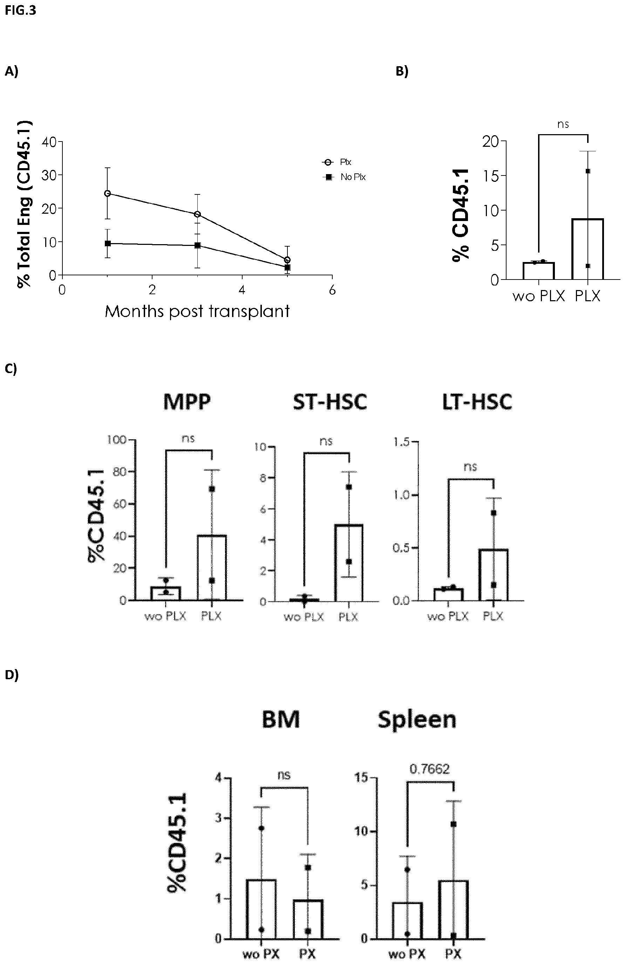

**Figure 3**: **Kinetics of hematopoietic engraftment in secondary recipients treated with Plerixafor plus Monoclonal Antibody conditioning.** A) Chimerism of CD45.1 cells until 5 months in WT peripheral blood, transplanted with 5-8x106 whole bone marrow of primary mice transplanted after treatment with or without PLX; B) Percentage of CD45.1 within LSK on secondary recipients; C) Chimerism of CD45.1 cells after 5 months within MPP, ST-HSC and LT-HSC immature progenitors. D) Chimerism of CD45.1 cells after 5 months in the whole BM and spleen. ns, non-significative.

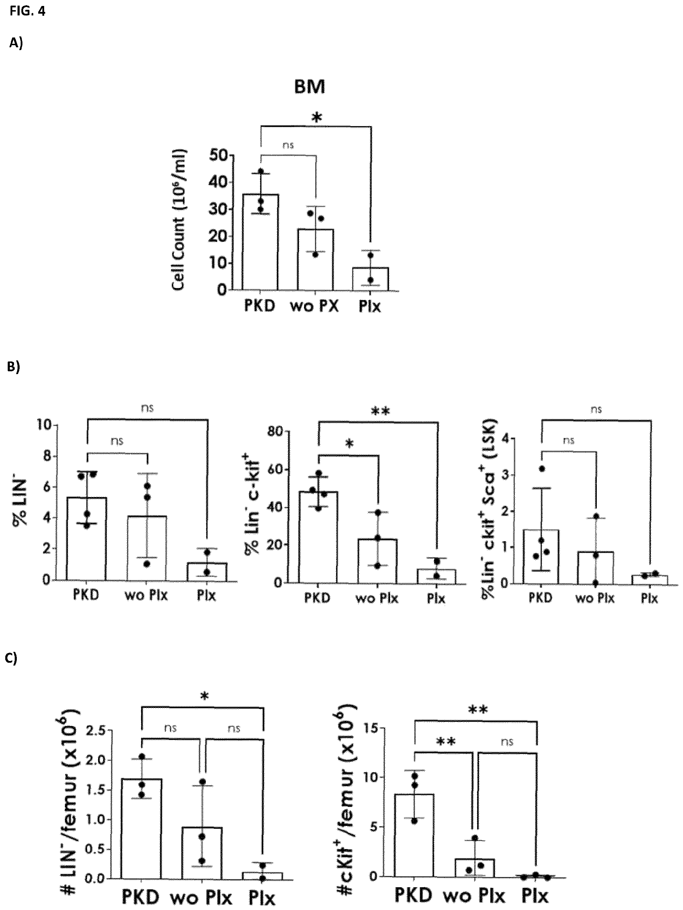

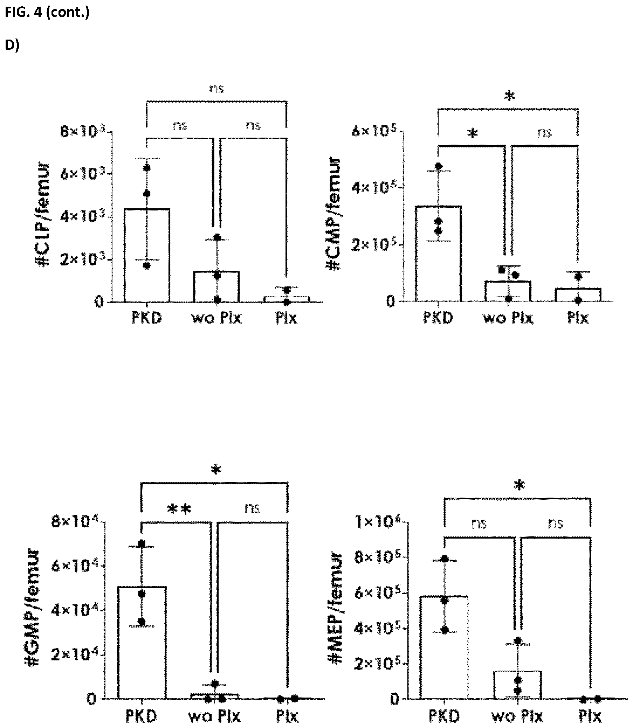

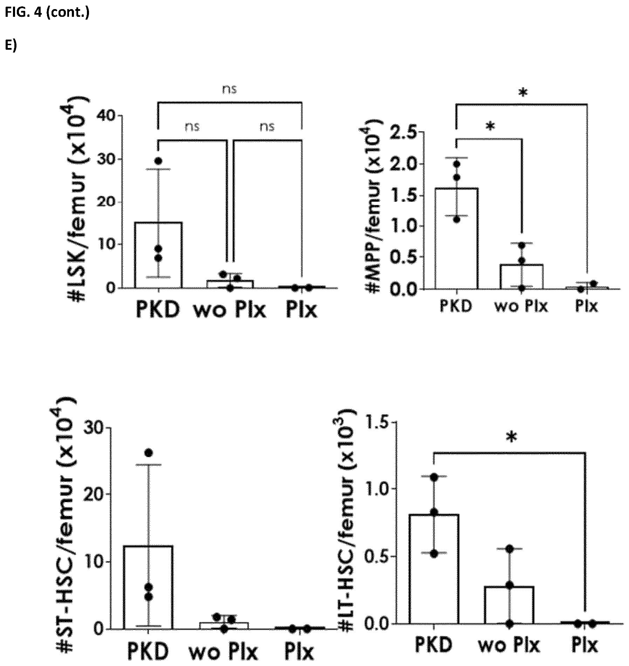

**Figure 4****: BM depletion after treatment with Monoclonal Antibody Cocktail plus Plerixafor conditioning.** A) Total BM cell counts in animals treated with the MoAb cocktail with (Plx) or without (wo Plx) Perixafor. Non-treated PKD animals (PKD) were also analyzed as controls; B) Percentage of non-mature cells (Lin-) and committed progenitors (Lin-c-kit+) in the BM of the animals; C) Total number per femur of non-mature cells (Lin-) and committed progenitors (Lin-c-kit+) in the BM of the animals: D) Total number per femur of lineage committed progenitors in the BM of the animals. CLP, common lymphoid progenitors; CMP, common myeloid progenitors; GMP, granulomacrophage progenitors; MEP, Megakaryocytic and erythroid progenitors; E) Total number per femur of more primitive progenitors and hematopoietic stem cells. LSK, Lineage-Sca-1+c-kit+; MPP, multipotent progenitors; ST-HSC, short-term hematopoietic stem cells; LT-HSC, Long-term hematopoietic stem cells. ns, non-significative, *p<0.05, **p<0.01.

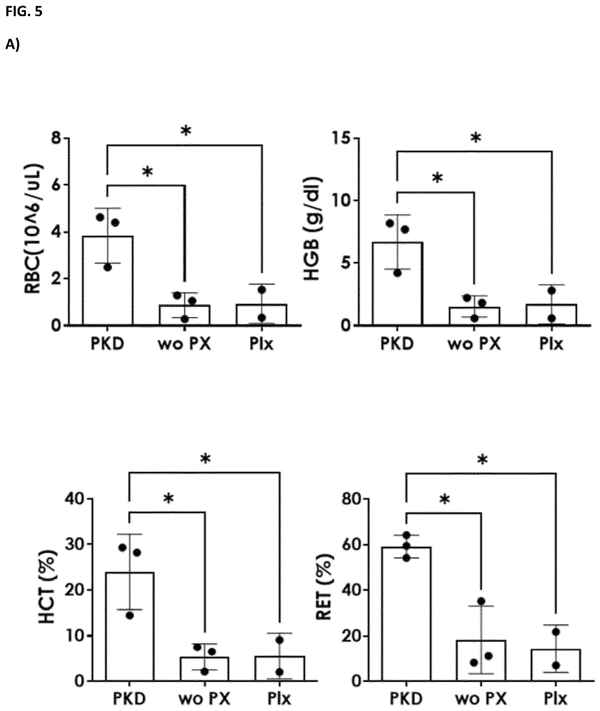

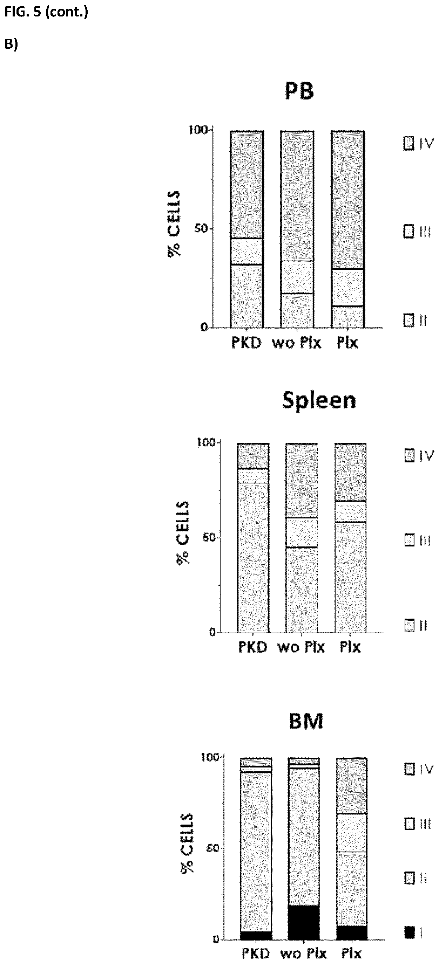

**Figure 5****: Mature erythroid lineage depletion after treatment with Plerixafor plus Monoclonal Antibody conditioning.** A) Peripheral red blood cell parameters in animals treated with the MoAb cocktail with (Plx) or without (wo Plx) Perixafor. Non-treated PKD animals (PKD) were also analyzed as controls. RBC, Red blood cells; HGB, Hemoglobin; HCT, Hematocrit; Ret, Reticulocytes. B) Analysis of erythroid compartments by flow cytometry in PB, spleen and bone marrow. I, proerythroblasts; II, Basophilic erythroblast; III, Orthochromatic erythroblasts; IV, reticulocytes and mature erythrocytes. *p<0.05.

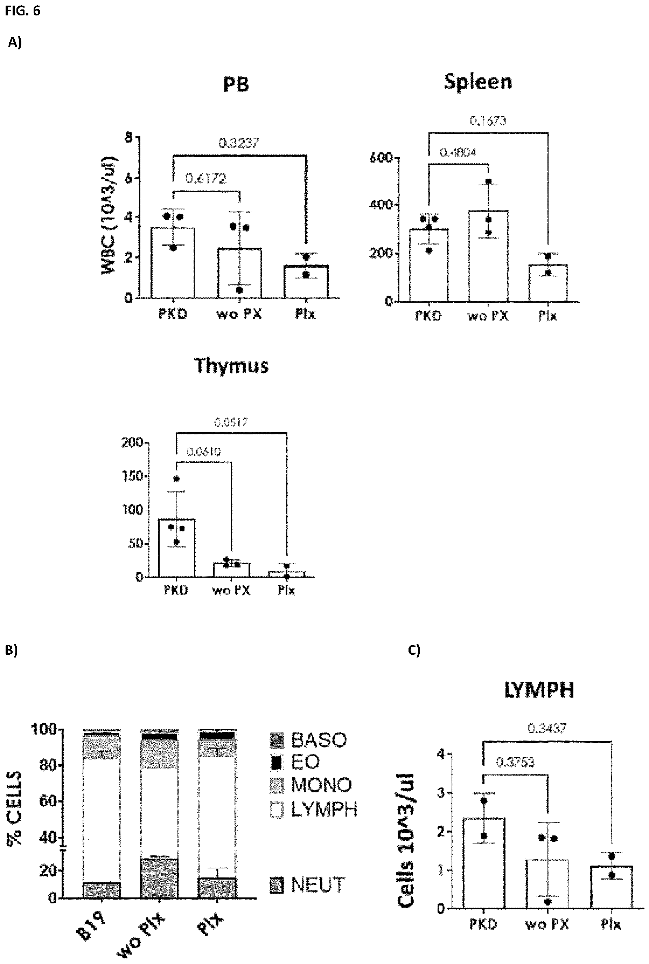

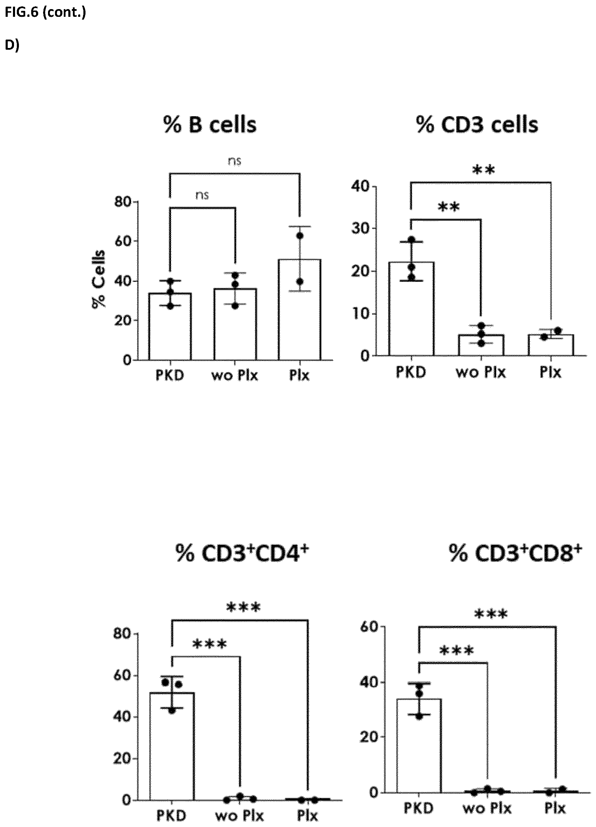

**Figure 6****. Peripheral blood cell subpopulations are depleted after treatment with Plerixafor plus Monoclonal Antibody conditioning.** A) Total white blood cells in peripheral blood (PB), spleen and thymus in animals treated with the MoAb cocktail with (Plx) or without (wo Plx) Perixafor. Non-treated PKD animals (PKD) were also analyzed as controls; B) Blood formula obtained with a hemacytometer. NEUT, Neutrophils; LYMPH, Lymphocytes; MONO, Monocytes; EO, Eosinophils; BASO, Basophils. C) Lymphocytes cell numbers in animals treated with the MoAb cocktail with (Plx) or without (wo Plx) Perixafor. D) Percentage of lymphoid subpopulations. ns, non-significative, **p<0.01, ***p<0.005.

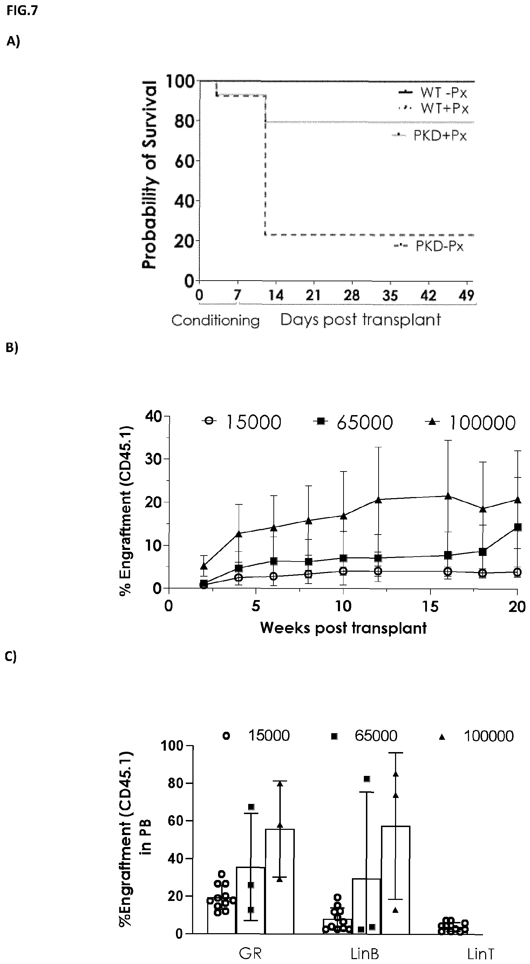

**Figure 7****. Hematopoietic conditioning with Monoclonal Antibody Cocktail plus Plerixafor increases survival rate and allows Long-Term Multi-Lineage Hematopoietic Reconstitution in an anemic mouse model of PKD.** A) Kaplan-Meyer survival representation of wild type and PKD mice after MoAb conditioning with and without Plx and transplanted with wild type CD45.1 congenic LSK cells; B) Hematopoietic chimerism in peripheral blood of PKD mice conditioned with MoAbs plus Plx and transplanted with different amounts of LSK cells (15,000; 65,000; and 100,000), periodically analyzed for a period of 20 weeks; C) Multilineage engraftment in MoAb plus Plx conditioned mice analyzed by chimerism in the lymphoid B (CD19+), lymphoid T (CD3+) and myeloid (Gr-1+Mac1+) differentiated cells.

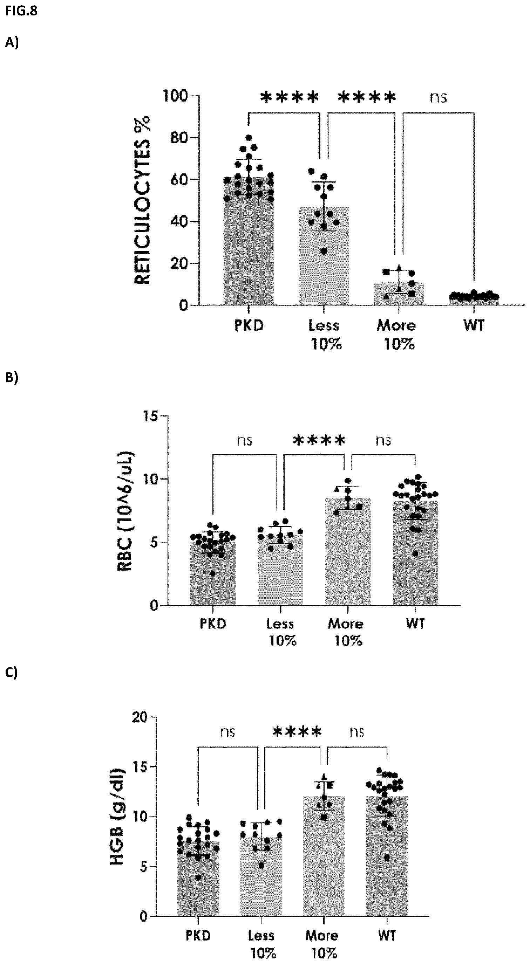

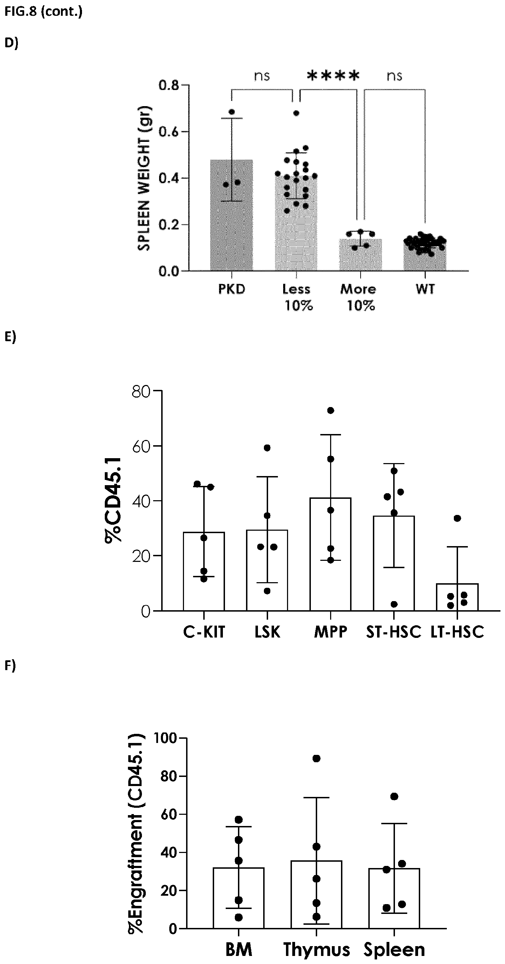

**Figure 8****. Reversion of PKD phenotype after non-genotoxic Plerixafor plus Monoclonal Antibody conditioning analyzed 6 months post-transplant.** A) Reticulocyte percentages in peripheral blood; Red bars, non-treated PKD mice; orange bars, non-genotoxic conditioned mice with and engraftment lower than 10%; blue bars, non-genotoxic conditioned mice with and engraftment higher than 10%; purple bar, wild type animals; each dot represents the analysis of single animal; represent the mean plus minus standard deviation. B) Red Blood Cell count in peripheral blood; groups as in A. C) Hemoglobin quantification (f/dL); groups as in A. D) Spleen weight; Groups as in A. E) level of engraftment in hematopoietic progenitors and more immature compartment of stem cells. C-KIT, Lineage-c-kit+ committed cells; LSK, Lineage-Sca-1+c-kit+ hematopoietic progenitors and stem cells; MPP, Multipotent progenitors; ST, Short-term hematopoietic stem cells; LT, Long-term hematopoietic stem cells. F) level of engraftment in hematopoietic organs after 6 months. ns, non-significative, ****p<0.001.

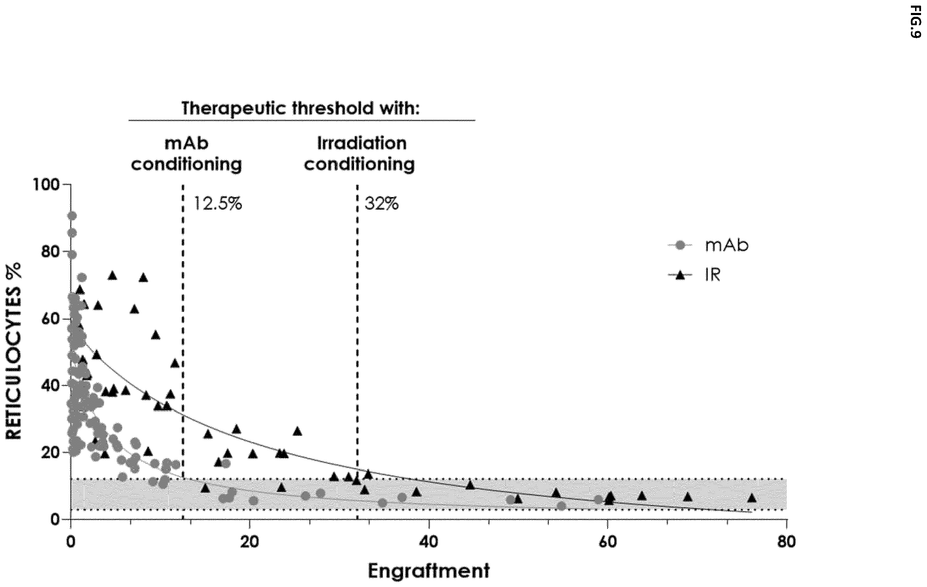

**Figure 9****. Minimum engraftment of wild type required to get reversion of anaemic phenotype.** Correlation between the percentage of reticulocyte and level of engraftment in peripheral blood, after non-genotoxic conditioning (red line) or genotoxic conditioning (black line). Each red dot represents a single blood reticulocyte analysis and engraftment correlation after infusion of wild type cells in PKD mice conditioned with the MoAb plus Plx treatment (non-genotoxic conditioning); Each black triangle represents a single blood reticulocyte analysis and engraftment correlation after infusion of wild type cells in PKD mice conditioned with x-ray (genotoxic conditioning). Gray square represents the area where normal levels are achieved.

### DESCRIPTION OF THE INVENTION

### Definitions

The terms "a," "an," or "the" as used herein not only include aspects with one member, but also include aspects with more than one member. For instance, the singular forms "a," "an," and "the" include plural referents unless the context clearly dictates otherwise. Thus, for example, reference to "a cell" includes a plurality of such cells and reference to "the agent" includes reference to one or more agents known to those skilled in the art, and so forth.

The term "administering or "administration" refers to the process by which agents, compositions, dosage forms and/or combinations disclosed herein are delivered to a subject for treatment or prophylactic purposes. Compositions, dosage forms and/or combinations disclosed herein are administered in accordance with good medical practices taking into account the subject's clinical condition, the site and method of administration, dosage, subject age, sex, body weight, and other factors known to the physician. For example, the terms "administering" or "administration" include providing, giving, dosing and/or prescribing agents, compositions, dosage forms and/or combinations disclosed herein by a clinician or other clinical professional.

The term "antibody" refers to a molecule comprising at least one immunoglobulin domain that binds to, or is immunologically reactive with, a particular target. The term includes whole antibodies and any antigen binding portion or single chains thereof and combinations thereof; for instance, the term "antibody" in particular includes bivalent antibodies and bivalent bispecific antibodies. A typical type of antibody comprises at least two heavy chains ("HC") and two light chains ("LC") interconnected by disulfide bonds. Each "heavy chain" comprises a "heavy chain variable domain" and a "heavy chain constant domain". The heavy chain constant domain typically comprises three constant domains, CH1, CH2, and CH3. Each "light chain" comprises a "light chain variable domain" and a "light chain constant domain". The light chain constant domain (CL) can be of the kappa type or of the lambda type. The VH and VL domains can be further subdivided into regions of hypervariability, termed Complementarity Determining Regions ("CDR"), interspersed with regions that are more conserved, termed "framework regions" ("FW"). Each VH and VL is composed of three CDRs and four FWs, arranged from amino-terminus to carboxy-terminus in the following order: FW1, CDR1, FW2, CDR2, FW3, CDR3, FW4.

As used herein, the term "antibody" encompasses intact polyclonal antibodies, intact monoclonal antibodies, bivalent antibody fragments (such as F(ab')2), multispecific antibodies such as bispecific antibodies, chimeric antibodies, humanized antibodies, human antibodies, and any other modified immunoglobulin molecule comprising an antigen binding site. An antibody can be of any the five major classes (isotypes) of immunoglobulins: IgA, IgD, IgE, IgG, and IgM, or subclasses thereof (e.g. IgG1, IgG2, IgG3, IgG4, IgA1 and IgA2), based on the identity of their heavy-chain constant domains referred to as alpha, delta, epsilon, gamma, and mu, respectively. The different classes of immunoglobulins have different and well-known subunit structures and three-dimensional configurations. Antibodies can be naked or conjugated to other molecules such as therapeutic agents or diagnostic agents to form immunoconjugates.

The term "pharmaceutical composition" refers to a composition that is physiologically acceptable and pharmacologically acceptable. In some instances, the composition includes an agent for buffering and preservation in storage, and can include buffers and carriers for appropriate delivery, depending on the route of administration.

The term "pharmaceutical acceptable carrier" refers to any and all solvents, dispersion media, coatings, antibacterial and antifungal agents, isotonic and absorption delaying agents, compatible with pharmaceutical administration. The use of such media and agents for pharmaceutically active substances is well known in the art. Acceptable carriers, excipients, or stabilizers are nontoxic to recipients at the dosages and concentrations employed and, without limiting the scope of the present invention, include: additional buffering agents; preservatives; co-solvents; antioxidants, including ascorbic acid and methionine; chelating agents such as EDTA; metal complexes (e.g., Zn-protein complexes); biodegradable polymers, such as polyesters; salt-forming counterions, such as sodium, polyhydric sugar alcohols; amino acids, such as alanine, glycine, glutamine, asparagine, histidine, arginine, lysine, ornithine, leucine, 2-phenylalanine, glutamic acid, and threonine; organic sugars or sugar alcohols, such as lactitol, stachyose, mannose, sorbose, xylose, ribose, ribitol, myoinisitose, myoinisitol, galactose, galactitol, glycerol, cyclitols (e.g., inositol), polyethylene glycol; sulfur containing reducing agents, such as glutathione, thioctic acid, sodium thioglycolate, thioglycerol, [alpha]-monothioglycerol, and sodium thiosulfate; low molecular weight proteins, such as human serum albumin, bovine serum albumin, gelatin, or other immunoglobulins; and hydrophilic polymers, such as polyvinylpyrrolidone. Other pharmaceutically acceptable carriers, excipients, or stabilizers, such as those described in Remington's Pharmaceutical Sciences 16th edition, Osol, A. Ed. (1980) may also be included in a pharmaceutical composition described herein, provided that they do not adversely affect the desired characteristics of the pharmaceutical composition.

The term "subject," herein are used herein interchangeably to include a human or animal. For example, the animal subject may be a mammal, a primate (e.g., a monkey), a livestock animal (e.g., a horse, a cow, a sheep, a pig, or a goat), a companion animal (e.g., a dog, a cat), a laboratory test animal (e.g., a mouse, a rat, a guinea pig, a bird), an animal of veterinary significance, or an animal of economic significance.

The term "treating" refers to an approach for obtaining beneficial or desired results including but not limited to a therapeutic benefit and/or a prophylactic benefit. By therapeutic benefit is meant any therapeutically relevant improvement in or effect on one or more diseases, conditions, or symptoms under treatment. For prophylactic benefit, the compositions may be administered to a subject at risk of developing a particular disease, condition, or symptom, or to a subject reporting one or more of the physiological symptoms of a disease, even though the disease, condition, or symptom may not have yet been manifested.

Unless defined otherwise, all technical and scientific terms used herein have the same meanings as commonly understood by one of ordinary skill in the art to which this technology belongs. Although exemplary methods, devices and materials are described herein, any methods and materials similar or equivalent to those expressly described herein can be used in the practice or testing of the present technology.

### Description of the invention

As detailed above one of the major drawbacks in the treatment of inherited hematopoietic diseases with transplantation of autologous genetically corrected cells, or with allogeneic healthy cells, is the requirement of hematopoietic conditioning of the patient to avoid endogenous competition and to generate available bone marrow niches to allow engraftment and reconstitution from the exogenous corrected (or healthy) cells. This hematopoietic conditioning is performed using chemotherapy with genotoxic agents, usually tightly controlled Busulfan dosage. This conditioning regime is very toxic with important side effects as described above. To allow the use of gene therapy as the first treatment option for anemias, and many other hematologic diseases, new less harmful conditioning regimens are required.

Therefore, a high need exists for finding alternative non-genotoxic conditioning regimen which will avoid side effects because the treatment is specific for the hematopoietic stem and progenitors' cells and not for other cell types or other tissues.

The inventors have therefore herein developed a new approach to find a solution to this problem by combining the monoclonal antibody treatment with the use of drug mobilizers with the idea of facilitating the interaction of the monoclonal antibodies with the hematopoietic stem and progenitors' cells when they are mobilized to the peripheral blood. With this strategy they have been able to achieve significantly higher exogenous engraftment with therapeutic efficacy, lower risk of engraftment failure and similar kinetics of hematopoietic recovery.

Firstly, the inventors could show that the combination of antibody conditioning and HSPC drug mobilizers increase engraftment in a wild type mouse model of autologous non-genotoxic conditioning (Example 1). The newly established conditioning treatment consists of three steps as described in Figure 1A and further detailed in the example. It was observed that the addition of Plx significantly increased engraftment in conditioned mice over time in comparison with MoAb alone treatment (Figure 1B). Data also showed no engraftment failure when Plx was added into the treatment vs 20% of engraftment failure with the MoAb alone treatment (Figure 1C). Importantly, normal recovery rates and mature lineage proportions were observed at 6 months post-transplantation (Figure 1D) and no differences were observed between the two approaches. In conditioned mice mobilized with Plx, engraftment in total Lineage negative (Lin⁻), c-Kit and LSK compartments was significantly higher compared to non-PIx treated mice (Figure 2B). Importantly, engraftment within the more immature hematopoietic stem cell compartment (Figure 2C) was also significantly increased in multipotent progenitors (MPP) and short-term HSCs (ST-HSC), and a trend was also observed in the long-term hematopoietic stem cell compartment phenotype (LT-HSC) (Figure 2D). Additionally, the use of Plx resulted in a higher engraftment in different hematopoietic organs such as thymus, bone marrow and spleen (Figure 2E).

The inventors could also observe that cells from mice previously mobilized with Plx maintained their engraftment capacity into secondary recipients (Figure 3A). Moreover, in secondary recipients transplanted with cells from primary recipients conditioned with the MoAb cocktail plus Plx, the engraftment in LSK and in more immature populations (MPP, ST-HSC and LT-HSC) was slightly increased compared to non-PIx treated mice (Figure 3B-C). The presence of CD45.1 cells from primary recipients was also detected 5 months after transplant in secondary recipients, although no differences were observed in the different hematopoietic organs for both conditioning treatments.

All together, these data indicate that the addition of the mobilizer Plx to the antibody-based conditioning improves engraftment efficiencies after transplantation both by reducing engraftment failure and increasing long term and stable hematopoietic engraftment rates in healthy donor settings.

The inventors then aimed to extend the approach to explore if the non-conditioning regime allowed an engraftment enough to correct the PKD phenotype. As further detailed in example 2 and shown in Figures 4 to 6, it could be shown that the combination of MoAbs plus Plerixafor depletes hematopoietic progenitors and stem cells in bone marrow of Pyruvate Kinase Deficiency mice.

It was further assessed whether the addition of Plx to the MoAb conditioning could reduce mortality associated with the treatment and allows exogenous engraftment in PKD mice. As explained in more detail in example 3, congenic wild type LSK cells were infused into the conditioned deficient mice to study the correction of the diseased phenotype. The mortality rate of animals treated with the MoAb combination alone decreased when Plx was added (Figure 7A). Moreover, when comparing the engraftment levels of C57BL/6 PKD mice to those of C57BL/6 wild-type mice, it was noticed that the wild type exhibited significantly higher engraftment levels. Engraftment levels were slightly increased when 100,000 cells compared with 15,000 cells transplanted (Figure 7B). The multilineage engraftment in MoAb plus Plx conditioned mice was analysed by assessing the chimerism in different cell lineages (Figure 7C) providing insights into the extent and durability of the engraftment of donor cells in different hematopoietic lineages and helping to evaluate the effectiveness of the conditioning treatment in achieving multilineage engraftment in this specific mouse model.

When studying whether the animals engrafted with wild-type cells reverted the anaemic phenotype as further detailed in example 4, it could be observed that a significant reduction in all parameters that reach normal levels in the group engrafted with more than 10% exogenous cells (Figure 8A-C). Normal levels were also observed in spleen weight in this group (Figure 8D). They conducted a more extensive analysis of engraftment in the reverted mice (Figure 8E and F). Engraftment was observed in all donor-derived stem cell compartments (Figure 8E) as well as in hematopoietic organs (Figure 8F). All together, these results demonstrate that the use of the combination of MoAb plus Plx proposed here is able to allow engraftment levels that allows a total and stable reversion of the anaemic PKD pathology.

To assess the minimum percentage of wild type engrafted cells required to achieve therapeutic levels in C57BL/6 PKD mice, the inventors focused on studying reticulocyte levels. Surprisingly, the correlation between reticulocyte levels and engraftment demonstrated that as little as 12.5% donor engraftment was sufficient to recover normal levels of reticulocytes (Figure 9 - red line). This therapeutic threshold was compared with the one observed using a conventional irradiation conditioning, observing that a recovery of reticulocyte levels was achievable with less engraftment levels when a non-genotoxic conditioning was applied (12.5%) than when a genotoxic one was applied (32%; Figure 9 - black line).

In summary, a significantly higher exogenous engraftment was observed compared to conditioning with the monoclonal antibody cocktail alone in wild type animals, in an autologous setting, transplanted with congenic cells. The combination regime when applied to a mouse model of Pyruvate Kinase Deficiency lead to bone marrow depletion. The infusion of congenic healthy cells was therapeutic, recovering the animals from all anaemic phenotype parameters. Most surprisingly it could be demonstrated that the use of the non-genotoxic conditioning described herein allows the recovery from the disease with levels of engraftment significantly lower than when genotoxic regimens were applied. This less aggressive conditioning treatment thus did not affect other non-hematopoietic cells that in turn could then help the engraftment of the exogenous healthy cells infused.

The inventors could thus convincingly show that the combination of both technologies, hematopoietic stem and progenitors' mobilization and depletion with monoclonal antibodies specific for these hematopoietic progenitors lead on the one hand to a significantly higher exogenous engraftment and on the other hand to the recovery from the disease with levels of engraftment that were significantly lower than when genotoxic regimens were applied. Whilst up to now, both strategies, treatments to mobilize hematopoietic stem and progenitors' cells from their bone marrow niches and specific elimination of these hematopoietic stem and progenitors' cells, have been applied separately, obtaining partial exogenous hematopoietic engraftments, the combination of both strategies facilitated the interaction of the monoclonal antibodies with the hematopoietic stem and progenitors' cells at the time they were mobilized to the peripheral blood.

The present invention therefore in one aspect relates to a composition comprising at least one monoclonal antibody directed against a protein expressed on hematopoietic cells for use in a method of providing stem cell engraftment in a subject, the method comprising at least one conditioning step that comprises simultaneously or subsequently administering to said subject
(i) the composition comprising at least one monoclonal antibody directed against a protein expressed on hematopoietic stem cells, and
(ii) at least one hematopoietic stem cell mobilization agent.

The present invention in a further aspect relates to a composition comprising at least one monoclonal antibody directed against a protein expressed on hematopoietic stem cells for use in a method of treatment of hereditary anemias, bone marrow failures, immunodeficiencies and metabolic diseases in a subject in need thereof, wherein the method of treatment comprises at least one conditioning step, wherein the conditioning step comprises the administration of the composition comprising at least one monoclonal antibody directed against a protein expressed on hematopoietic stem cells and the simultaneous or subsequent administration of at least one hematopoietic stem cell mobilization agent to the subject.

As used herein the term "hereditary anemias" generally refers to hereditary diseases that cause a reduction in the number or functionality of the red blood cells in a subject which can result in anemia and can require sporadic or regular red blood cell transfusions.

Simultaneous administration refers to substantially simultaneous administration meaning that the composition comprising at least one monoclonal antibody directed against a protein expressed on hematopoietic cells and the hematopoietic stem cell mobilization agent can be administered in the same pharmaceutical composition, or immediately after each other in two separate pharmaceutical compositions.

Subsequent administration refers to the administration of the composition comprising at least one monoclonal antibody directed against a protein expressed on hematopoietic stem cells and the hematopoietic stem cell mobilization agent one after the other. The time between first and second administration can be from about 5 min to 3 h, from about 20 min to 2 min, from about 40 min to 1.5h, from about 50 min to 1h.

In one embodiment the administration can be repeated, i.e., after the first and second administration (one administration cycle), a second administration cycle again comprising a first and a second administration in the same order as the first administration cycle can be added. In this embodiment in the first cycle the second administration can be done between 1 min and 2 h after the first administration and the second cycle started the latest after 24h of the first cycle.

It is also foreseen, that if regarded necessary, a third and fourth administration cycle may be added.

In one embodiment, the mobilization agent, such as for examples G-CSF, can be administered up to 5 to 7 days after the at least one monoclonal antibody.

In one embodiment the mobilization agent, such as for example BIO5192, can be administered up to 1 to 2 days after the at least one monoclonal antibody.

During subsequent administration the hematopoietic stem cell mobilization agent can be administered first and the composition comprising at least one monoclonal antibody directed against a protein expressed on hematopoietic stem cells second, or vice versa. Preferably the hematopoietic stem cell mobilization agent is administered first and the composition comprising at least one monoclonal antibody directed against a protein expressed on hematopoietic cells second.

In one preferred embodiment of present invention, the administration is done subsequently with from about 40 min to 1.5h, from about 50 min to 1h between first and second administration. It has been found that in the mouse model the administration of the antibody at one hour after the mobilization was preferred, as at that timepoint the mobilization of the cells was at its peak. The benefit of such an administration is the facilitation of the interaction of the monoclonal antibodies with the hematopoietic stem and progenitors' cells directly at the time point when they are mobilized to the peripheral blood.

In one preferred embodiment the administration is done simultaneously.

### DEPLETION

Depletion regimes serve to reduce or eliminate endogenous HSPCs. Endogenous HSPCs can be reduced by a factor of e.g., at least 10%, 25%, 50%, 75% or 90% before introducing replacement HSPCs. Some regimes do not reduce endogenous HSPCs by more than, e.g., 90%, 75%, 50%, 25% or 10% before introducing replacement HSPCs. Some regimes reduce endogenous HSPCs by 25-75% or 25-95%. Depletion regimes can also be defined by corresponding percentage reductions of HSCs or HPCs, the constituent cells of HSPCs. Because both express anti-c-kit, both can undergo deletion to a similar extent.

Depletion regimes generally involve administration of an antibody specifically binding to c-kit (CD117) (e.g. WO 2008067115) or other agents binding to c-kit. CD117 is a receptor tyrosine kinase type III, which binds to stem cell factor (SCF), also known as "steel factor" or "c-kit ligand". When CD117 binds to SCF it forms a dimer that activates its intrinsic tyrosine kinase activity, that in turn phosphorylates and activates signal transduction molecules that propagate the signal in the cell. CD117 is an important cell surface marker used to identify certain types of hematopoietic (blood) progenitors in the bone marrow. Hematopoietic stem cells (HSC), multipotent progenitors (MPP), and common myeloid progenitors (CMP) express high levels of CD117.

A number of antibodies that specifically bind human CD117 are known in the art and commercially available, including without limitation SR1, 2B8, ACK2, YB5-B8, 57A5, 104D2, etc. (see US20180214525). Of interest is the humanized form of SR1, AMG 191, described in US Patent no. 8,436, 150, and 7,915,391 which is an aglycosylated IgG 1 humanized antibody. Further anti-c-kit immunotherapeutic agents that can be used in the present invention include FSI-174 (FortySeven, Inc.), and CDX-0158 or CDX-0159 (Celldex Therapeutics, Inc.) as well as inhibitors of c-kit described in the following publications: WO199203459, WO199221766, WO2007127317, WO2008115300, WO2012154480, WO2019155067, and WO2020076105.

Any of these antibodies, including chimeric, veneered or humanized forms, or antibodies binding the same epitope or competing therewith for binding to c-kit can be used in the herein disclosed methods.

An effective dose of an anti-CD117 antibody may be administered in one or more doses, including a single dose, which may be at least about one week, i.e., 7 days, prior to transplantation, at least about 6 days, at least about 5 days prior to transplantation, at least about 4 days, at least about 3 days prior to transplantation.

In a preferred embodiment the anti-CD117 is given 5 or 6 days prior to transplantation.

The period of time between dosing and transplantation is sufficient to substantially eliminate the anti-CD117 antibody from the circulation of the recipient. For example, the decrease in peak serum levels following administration is usually the time sufficient for the level to decrease as least about 10-fold from peak levels, usually at least about 100-fold, 1000-fold, 10,000-fold, or more. It is preferable to introduce the donor stem cells within the empty niche "window" following the wash-out period, usually within about 3 days, about 2 days, about 1 day, or at the time of clearance.

In some embodiments, an effective dose of an anti-CD117 antibody is up to about 10 mg/kg, up to about 5 mg/kg; up to about 1 mg/kg, up to about 0.5 mg/kg; up to about 0.1 mg/kg; up to about 0.05 mg/kg; where the dose may vary with the specific antibody and recipient.

The depletion regime can also include an immunotherapeutic agent inhibiting CD47-SIRPα interaction for use in combination with an antibody against c-kit (see WO2016033201). Such an agent promotes effector-mediated elimination of endogenous HSPCs mediated by anti-c-kit. CD47 is also known as any of IAP, MERG, and 0A3. Human CD47, which is targeted by immunotherapeutic agents in treatment of humans, has been assigned exemplary accession numbers NCBI Gene ID: 961 and UniProt Q08722.

As used herein, the term "anti-CD47 agent" or "agent that provides for CD47 blockade" refers to any agent that reduces the binding of CD47 (e.g., on a target cell) to SIRPa (e.g., on a phagocytic cell). Non-limiting examples of suitable anti-CD47 reagents include SIRPa reagents, including without limitation high affinity SIRPa polypeptides, anti- SIRPa antibodies, soluble CD47 polypeptides, and anti-CD47 antibodies or antibody fragments. In some embodiments, a suitable anti-CD47 agent (e.g. an anti-CD47 antibody, a SIRPa reagent, etc.) specifically binds CD47 to reduce the binding of CD47 to SIRPa.

Examples of suitable anti-CD47 antibodies for use in the methods of present invention include clones B6H12, 5F9, 8B6, C3, (for example as described in WO2011/143624) CC9002 (Vonderheide, Nat Med 2015; 21: 1122-3, 2015), and SRF231 (Surface Oncology). Suitable anti-CD47 antibodies include human, humanized or chimeric versions of such antibodies, antibodies binding to the same epitope or competing therewith for binding to CD47. Humanized antibodies (e.g., hu5F9-lgG4-WO2011/143624) are especially useful for in vivo applications in humans due to their low antigenicity. Direct contact residues for hu5F9-IgG4 in human CD47 have been reported to be K39, K41, E97, T99 and E104 (LC) and E29, R103 and E104 (HC) (Weiskopf et al., J. Clin. Invest 126, 2610-262-(2016)). Other examples of immunotherapeutic agents against CD47 inhibiting its interaction with SIRPα include anti-CD47 mAbs (Vx-1004), anti-human CD47 mAbs (CNTO-7108), CC-90002, CC-90002-ST-001, NI-1701, NI-1801, RCT-1938, ALX-148, RRx-001, DSP-107, VT-1021, TTI-621, TTI-622, IMM-02 SGN-CD47M.

In one embodiment of present invention the at least one monoclonal antibody directed against a protein expressed on hematopoietic cells is selected from an anti CD47, an anti c-kit (anti CD117), an anti-CD45, an anti-CD135, an anti-CD34, an anti CD4, an anti CD3 and an anti CD38 antibody, or a combination thereof.

In a preferred embodiment the monoclonal antibody used is anti CD47, anti c/kit, or a combination thereof.

The effective dose of an anti-CD47 agent can vary with the agent, but will generally range from up to about 50 mg/kg, up to about 40 mg/kg, up to about 30 mg/kg, up to about 20 mg/kg, up to about 10 mg/kg, up to about 5 mg/kg; up to about 1 mg/kg, up to about 0.5 mg/kg; up to about 0.1 mg/kg; up to about 0.05 mg/kg; where the dose may vary with the specific antibody and recipient. Agents that bind to CD47, e.g. soluble SIRPa polypeptides and anti- CD47 antibodies, may be administered at higher doses due to the larger number of CD47 expressing cells in the body.

The anti-CD47 agent may be administered one or a plurality of days prior to transplantation, and in some embodiments is administered daily for a period of from about 1, about 2, about 3, about 4, about 5, about 6, about 7 or more days, i.e. from about 1 to 7 days, from about 1 to 5 days, from about 1 to 3 days, etc.

In a preferred embodiment the "anti-CD47 agent" is an anti-CD47 antibody. In a preferred embodiment it is given 7 days prior to transplantation followed by a daily administration from day 5 to day 2 prior to transplantation.

As with the anti-c-kit agent, targeting CD47 can affect the donor stem cells after infusion, and therefore a wash-out period is required before infusion of hematopoietic cells. The washout period may be shorter than with the c-kit antibody, but is typically at least about 24 hours, at least 36 hours, at least 48 hours, and may be up to about one week, up to about 5 days, up to about 3 days, etc.

Immunotherapeutic agents, including antibodies and Fc fusion proteins, are administered in a regime effective to achieve the desired purpose of reducing or eliminating endogenous HSPCs. An effective regime refers to a combination of dose, frequency of administration and route of administration. A regime of an antibody specifically binding to c-kit preferably delivers sufficient antibody for substantial saturation of c-kit receptors on the target population of HSPC's expressing c-kit but not a vast excess over such an amount resulting in unnecessary long persistence of anti-c-kit after administration, which may result in delay in administering replacement HSPC's or unintended killing of replacement of HSPC's. It has been found that a total amount of anti-c-kit of about 0.15-2 mg/kg is suitable for such purposes. This amount can be administered as a single dose, two doses, or three or more dosages. One regime involves administering a single dose of e.g., 0.15-1 mg/kg, 0.25-1 mg/kg, 0.25-0.5 mg/kg, or 0.3 mg/kg anti-c-kit. Another regime involves administering two doses of e.g., 0.15-1 mg/kg, 0.25-1 mg/kg, 0.25-0.5 mg/kg, or 0.3 mg/kg anti-c-kit spaced by 3-7 days. Another regime involves administering three or more doses of 0.15-1 mg/kg, 0.25-1 mg/kg, 0.25-0.5 mg/kg, or 0.3 mg/kg anti-c-kit, optionally spaced by 10-30 days.

Dosage provided are for antibodies to c-kit, particularly any of the humanized SR1 antibodies as described above. These dosages also provide guidance for other immunotherapeutic agents; however, dosages of such agents can be adjusted for differences in molecular weight and/or binding affinity to achieve substantially the same level of reduction of HSPCs expressing c-kit as obtained for humanized SR1.

Immunotherapy agents inhibiting CD47-SIRPα are administered in a regime effective to promote reduction of HSPCs expressing c-kit by an immunotherapeutic agent specifically binding to c-kit (anti-c-kit). Anti-c-kit may or may not effect reduction of HSPCs expressing c-kit in the absence promotion by an immunotherapy agent inhibiting CD47-SIRPα. Exemplary doses for immunotherapy agents inhibiting CD47-SIRPα are at least any of 0.05 mg/kg, 0.1 mg/kg, 0.5 mg/kg, 1 mg/kg up to any of 5 mg/kg, 10 mg/kg, 20 mg/kg, 30 mg/kg 40 mg/kg or 50 mg/kg. Some exemplary ranges are 0.05 mg/kg-50 mg/kg, 0.1 mg/kg to 20 mg/kg, 1 mg/kg to 10 mg/kg, or 10 mg/kg to 30 mg/kg. Optionally, such immunotherapeutic agents, particularly those specifically binding to CD47, can be administered initially at one or more priming doses, followed by one or more therapeutic doses to reduce undesired crosslinking of red blood cells, as described by e.g., WO2017181033. A preferred regime is a priming dose at 0.5 to 5 mg/kg, e.g., 1 mg/kg following by a therapeutic dose of 10-30 or 15-20 mg/kg. The therapeutic dose is administered e.g., 3-15 or 5-10 or 7 days after the priming dose.

Combination or co-administration treatment with anti-c-kit and an immunotherapeutic agent inhibiting CD47-SIRPα involves administering the respective agents sufficiently proximal in time for the latter to promote reduction of HSPCs expressing c-kit by the former. Typically in combination regimes both agents are present at detectable levels in subject serum at the same time. In some combination regimes, a priming dose of an immunotherapeutic agent inhibiting CD47-SIRPα is administered followed by administration of a dose of anti-C-kit and a therapeutic dose of the immunotherapeutic agent inhibiting CD47-SIRPα at the same time. In some such regimes, the two agents are administered simultaneously by co-infusion. In some regimes, multiple doses of an immunotherapeutic agent specifically binding to c-kit and multiple doses of an immunotherapeutic agent inhibiting CD47-SIRPα are administered, optionally in a pairwise manner with one dose of each being administered on the same day. Such regimes can be preceded by a priming dose of the immunotherapeutic agent inhibiting CD47-SIRPα.

Combination treatment with an immunotherapeutic agent specifically binding to c-kit and an immunotherapeutic agent specifically binding to CD47 or SIRPα can be performed in further combination with one or more agents effective to deplete other cells of the immune system. For example, MCL1 apoptosis regulator, BCL2 family member (MCL1) inhibitors can be used to ablate NK cell. In various embodiments, an ablation regime as described herein, is combined with an inhibitor of MCL1 apoptosis regulator, BCL2 family member (MCL1, TM; EAT; MCL1L; MCL1S; Mcl-1; BCL2L3; MCL1-ES; bcl2-L-3; mcl1/EAT; NCBI Gene ID: 4170). Examples of MCL1 inhibitors include AMG-176, AMG-397, S-64315, AZD-5991, 483-LM, A-1210477, UMI-77, JKY-5-037, APG-3526 and those described in WO2018183418, WO2016033486, and WO2017147410.

Immunotherapeutic agents are typically administered as pharmaceutical compositions in which the agent is combined with one or more pharmaceutically acceptable carriers. A variety of aqueous carriers can be used, e.g., buffered saline and the like. These solutions are sterile and generally free of undesirable matter. These compositions may be sterilized by conventional techniques. The compositions may contain pharmaceutically acceptable auxiliary substances as required to approximate physiological conditions such as pH adjusting and buffering agents, toxicity adjusting agents and the like, e.g., sodium acetate, sodium chloride, potassium chloride, calcium chloride, sodium lactate and the like. The concentration of active agent in these formulations can vary widely, and is selected primarily based on fluid volumes, viscosities, body weight and the like in accordance with the particular mode of administration selected and the patient's needs (e.g., Remington's Pharmaceutical Science (15th ed., 1980) and Goodman & Gillman, The Pharmacological Basis of Therapeutics (Hardman et al., eds., 1996).

### IMMUNOSUPPRESSION

In one embodiment of present invention the conditioning step further comprises administering at least one agent that induces transient immunosuppression in said subject.

In some embodiments the at least one agent that induces transient immunosuppression is suitable for depleting one or both of T lymphocytes and natural killer (NK) cells. Agents that deplete T cells specifically include without limitation, agents, including antibodies, specific for CD3, CD4, CD8, etc. Agents that deplete T cells and NK cells include without limitation, agents, including antibodies, specific for CD2, CD52, CD45, anti-thymocyte globulin (ATG), etc. Agents that deplete NK cells specifically include without limitation, agents, including antibodies, specific for CD122, CD56, etc. The depleting agent(s) can be administered prior to infusion of the exogenous stem cells, and are optionally active after infusion, so long as the targeted cells have been depleted when the exogenous stem cells are administered.

In a preferred embodiment of present invention, the at least one agent that induces transient immunosuppression is selected from the groups consisting of agents that inhibit CD4, CD8, CD122 and CD40L, such as for example anti CD4, anti CD8, anti CD122 and anti CD40L antibodies, or combinations thereof.

In a further embodiment the at least one agent that induces transient immunosuppression is selected from the mycophenolic acid, cyclosporine A, rapamycin, FK506, and corticosteroids.

### HSPCS AND THEIR MOBILIZATION

Depending on the application, the HSPCs to be introduced into a subject can be autologous (from that subject), allogenic (from another individual of the same species), or xenogenic (from a different species). If allogenic, the HSPCs can be matched fully or partially or unmatched for MHC alleles.

Although all HSPCs are capable of propagation and differentiation into cells of myeloid or lymphoid linages or both, HSPCs include cells at different stages of differentiation. Primitive stem cells can propagate indefinitely and form all cell types of myeloid and lymphoid lineages. Primitive stem cells differentiate into multi-potent progenitors, which can give rise to all cells of both myeloid and lymphoid lineages but cannot propagate indefinitely. Multipotent progenitors give rise to oligo-potent progenitors including the common lymphoid progenitor, CLP, which gives rise to mature B lymphocytes, T lymphocytes, and natural killer (NK) cells. Multipotent progenitors also give rise to the common myeloid progenitor (CMP) which further differentiates into granulocyte-macrophage progenitors, which differentiate into monocytes/macrophages and granulocytes, and megakaryocyte/erythrocyte progenitors, which differentiate into megakaryocytes/platelets and erythrocytes (see FIG. 1 of Bryder et al., Am. J. Pathol. 169, 338-346 (2006)).

HSPCs can be obtained by harvesting from bone marrow, from peripheral blood or umbilical cord blood. Bone marrow is generally aspirated from the posterior iliac crests while the donor is under either regional or general anesthesia. Additional bone marrow can be obtained from the anterior iliac crest. Mobilization of stem cells from the bone marrow into peripheral blood can be achieved by cytokines such as G-CSF, GM-CSF or Plerixafor (also known as AMD3100 or Mozobil).

In one embodiment of present invention the hematopoietic stem cell mobilization agent is selected from the group consisting of Plerixafor, G-CSF, GM-CSF, GM-CSF, BIO5192, or combinations thereof.

In a preferred embodiment the hematopoietic stem cell mobilization agent is Plerixafor.

HSPCs can also be obtained from umbilical cord blood typically for allogenic transplant. UCB is enriched in primitive stem/progenitor cells able to produce in vivo long-term repopulating stem cells.

Blood cells isolated from these procedures can undergo enrichment for HSPCs or a subset thereof, e.g., primitive stem cells and/or common progenitor by affinity enrichment for characteristic cell surface markers. Such markers include CD34; CD90 (thy-1); CD59; CD1 10 (c-mpl); c-kit (CD-117). Cells can be selected by affinity methods, including magnetic bead selection, flow cytometry, and the like from the donor hematopoietic cell sample. Several immunoselection devices, including Ceparte, Isolex 300i, and CliniMACS are commercially available for CD34+ cell selection.

The HSPC composition can be at least about 50% pure, as defined by the percentage of cells that are CD34+ in the population, may be at least about 75% pure, at least about 85% pure, at least about 95% pure, or more.

### DISEASES AND TREATMENT

Hematopoietic stem and progenitors' cell (HSPCs) transplant of healthy cells is currently the only curative treatment for diverse diseases affecting the blood system, including hereditary anemias, bone marrow failures, immunodeficiencies and metabolic diseases.

The methods of present invention provide an improved method of treatment to these disorders by increasing the engraftment efficiency. Furthermore, it could surprisingly be shown that already with lower engraftment a phenotype reversion could be observed compared with other genotoxic conditioning treatments.

In one embodiment of present invention the hereditary anemias are selected from B-thalassemia, sickle cell disease (SCD) or pyruvate kinase deficiency (PKD).

In a preferred embodiment the hereditary anemia is pyruvate kinase deficiency.

Pyruvate kinase deficiency (PKD) is the most common erythroid inherited enzymatic defect causing chronic non-spherocytic hemolytic anemia. The prevalence of PKD is estimated at 1-9 cases per 100,000 people in the Caucasian population. PKD is an autosomal recessive disorder caused by mutations in the *PKLR* gene. This gene encodes for two different transcript variants, RPK and LPK, expressed in red blood cells and liver respectively. To date, more than 200 different mutations in the *PKLR* gene have been linked to PKD. Therapy options for PKD are palliative and include regular red blood cell transfusion, splenectomy and iron chelation therapy. So far, allogeneic hematopoietic stem cell transplant (HSCT) represents the only curative treatment for severely affected patients. Autologous HSCT of genetically corrected cells, also called hematopoietic stem and progenitor cell (HSPC) gene therapy, is being used to treat many blood cell genetic diseases.

As could be shown specifically for PKD in the examples, significantly higher exogenous engraftment with therapeutic efficacy, lower risk of engraftment failure and similar kinetics of hematopoietic recovery could be observed when applying the methods of present invention.

In a preferred embodiment the method of present invention is particularly suitable for the treatment of pyruvate kinase deficiency.

In one embodiment the bone marrow failures are selected from Fanconi anemia (FA), Blackfan-Diamond anemia (BDA), dyskeratosis congenita (DC), Shwachman-Diamond syndrome (SDS), congenital amegakaryocytic thrombocytopenia (CAMT) or reticular dysgenesis (RD).

In one embodiment the immunodeficiencies are selected from adenosine deaminase (ADA) deficiency, X-linked severe combined immunodeficiency (SCID), Wiskott Aldrich or X-linked chronic granulomatous disease (X-CGD).

In one embodiment the metabolic diseases are selected from metachromatic leukodystrophy or Mucopolysaccharidosis 1 or 3.

### EXAMPLES

### Example 1: Combination of antibody conditioning and HSPC drug mobilizers increase engraftment in wild type (C57BL/6) model of autologous non-genotoxic conditioning

To study the combination of hematopoietic stem cell mobilizers and monoclonal antibodies as new conditioning treatment for HSCT, we established the conditioning treatment described in Figure 1A. This conditioned treatment is divided in three different steps. In the first one, specific antibodies, anti-CD117 (c-kit) (ACK2) to deplete mouse hematopoietic stem, and anti-CD47 to block a dominant anti-phagocytic signal ("don't eat me" signal) were used. The second step involved immunosuppression using anti-CD122 to deplete host NK cells, anti-CD40-CD40L to further inhibit T cell-mediated rejection, and anti-CD4/anti-CD8 to reduce possible host rejection. The third step focused on the addition of the HSPC drug mobilizer, Plx, with the idea of removing the HSPCs from the bone marrow to make them more accessible to the antibody treatment. The experimental procedure consisted in the use of HSPC from CD45.1 donors to be infused in conditioned CD45.2 recipients of the C57BL/6 strain. Fifteen thousand lineage negative, c-kit positive and Sca-1 positive (LSK) cells were transplanted into conditioned mice and engraftment was determined by the presence of more than 1% of donor cells in peripheral blood lineages, measured periodically through blood sampling and flow cytometry. The addition of Plx significantly increased engraftment in conditioned mice over time in comparison with MoAb alone treatment (Figure 1B). Data also showed no engraftment failure when Plx was added into the treatment vs 20% of engraftment failure with the MoAb alone treatment (Figure 1C). Importantly, normal recovery rates and mature lineage proportions were observed at 6 months post-transplantation (Figure 1D) and no differences were observed between the two approaches.

Upon sacrifice at 6 months' post-transplantation, the engraftment of donor-derived LT-HSCs compartments were analysed as described in figure 2A. In conditioned mice mobilized with Plx, engraftment in total Lineage negative (Lin⁻), c-Kit and LSK compartments was significantly higher compared to non-PIx treated mice (Figure 2B). Importantly, engraftment within the more immature hematopoietic stem cell compartment (Figure 2C) was also significantly increased in multipotent progenitors (MPP) and short-term HSCs (ST-HSC), and a trend was also observed in the long-term hematopoietic stem cell compartment phenotype (LT-HSC) (Figure 2D). Additionally, the use of Plx resulted in a higher engraftment in different hematopoietic organs such as thymus, bone marrow and spleen (Figure 2E).

To evaluate the long-term engraftment potential of the transplanted cells, secondary transplants were performed. Recipient mice (transgenic C57BL/6 mice ubiquitously expressing the Red Fluorescent Protein) were irradiated with 9 Gy (two doses of 4.5 Gy, 24 hours apart) prior to transplantation. We transplanted 5 to 8 million whole bone marrow cells from primary non-genotoxic conditioned mice. Percentage of engraftment in peripheral blood and various hematopoietic organs were analysed over time. We observed that cells from mice previously mobilized with Plx maintained their engraftment capacity into secondary recipients (Figure 3A). Moreover, in secondary recipients transplanted with cells from primary recipients conditioned with the MoAb cocktail plus Plx, the engraftment in LSK and in more immature populations (MPP, ST-HSC and LT-HSC) was slightly increased compared to non-PIx treated mice (Figure 3B-C). The presence of CD45.1 cells from primary recipients was also detected 5 months after transplant in secondary recipients, although no differences were observed in the different hematopoietic organs for both conditioning treatments. All together, these data indicate that the addition of the mobilizer Plx to the antibody-based conditioning improves engraftment efficiencies after transplantation both by reducing engraftment failure and increasing long term and stable hematopoietic engraftment rates in healthy donor settings.

### Example 2: The combination of MoAbs plus Plerixafor depletes hematopoietic progenitors and stem cells in bone marrow of Pyruvate Kinase Deficiency mice.

Once we established that the combination of Plx and conditioned antibodies led to increased engraftment in wild type C57BL/6 mice, we aimed to extend this approach to explore if the non-conditioning regime allowed an engraftment enough to correct the PKD phenotype. We observed that our PKD mouse model showed significant differences in bone marrow composition compared to wild type C57BL/6 mice. We investigated whether the mobilization profile with Plx differed in these mice. Mice treated with or without Plx were sacrificed on day 0 and their bone marrow was analysed as above (see Figure 1A). We found that the use of 6 antibodies resulted in a reduction in white blood cell numbers, but this decrease was bigger with the addition of Plx (Figure 4A). Furthermore, we observed a decrease in c-Kit and LSK cell compartments (Figure 4B). We also observed a higher decrease in absolute number in Lin⁻, Lin⁻c-Kit⁺ progenitors and LSK pool of stem cells in the conditioned treatment plus Plx (Figure 4C). Moreover, similar differences were obtained within committed progenitors (Figure 4D) and in the most immature phenotypes (Figure 4E).

We analysed the erythroid compartment in the different groups of animals. Erythroid parameters in peripheral blood were affected similarly by both conditions (Figure 5A). We also found a decrease in late erythroid progenitors, that was more remarkable when Plx was added to the non-conditioning regime (Figure 5B).

As for other mature hematopoietic lineages, we observed a slight decrease in the number of total nucleated cells in the peripheral blood and thymus (Figure 6A and B) when Plx was added. Interestingly, the number of total cells in spleen showed an opposite trend between the two conditions (Figure 6A).

As mentioned above, the second step of the conditioning treatment is related to immunosuppression. In this regard, we analysed the percentage of lymphoid cells in peripheral blood. We observed a slightly decrease in terms of absolute number of lymphoid cells, corresponding to a significant reduction in the CD3⁺ compartment (Figure 6C). Further analysis of CD3⁺ cells revealed a significant reduction in both CD4⁺ and CD8⁺ cells. As expected, there were no significant changes in the percentage of lymphoid B cells (figure 6D).

### Example 3: The addition of Plx to the MoAb conditioning reduces mortality associated with the treatment and allows exogenous engraftment in PKD mice.

After demonstrating the hematopoietic depletion caused by the treatment in PKD mice, congenic wild type LSK cells were infused into the conditioned deficient mice to study the correction of the diseased phenotype. Unexpectedly, we observed an increased mortality rate when the animals were treated with the MoAb combination alone, which decreased when Plx was added (Figure 7A). Moreover, when comparing the engraftment levels of C57BL/6 PKD mice to those of C57BL/6 wild-type mice, it was notice that the wild type exhibited significantly higher engraftment levels. We had reported a considerable reduction in engraftment levels in this mouse model due to the severe anaemic phenotype. Additionally, when comparing the engraftment levels of C57BL/6 PKD mice with and without Plx treatment, the few mice that survived without Plx demonstrated engraftment levels similar to those obtained when Plx was administered.

To enhance engraftment levels in C57BL/6 PKD mice, we tested different graft sizes (15,000, 65,000, and 100,000). Engraftment levels were slightly increased when we transplanted 100,000 cells compared with 15,000 cells (Figure 7B). The multilineage engraftment in MoAb plus Plx conditioned mice was analysed by assessing the chimerism in different cell lineages (Figure 7C), including lymphoid B cells (CD19+), lymphoid T cells (CD3+), and myeloid cells (Gr-1+Mac1+), derived from CD45.1 cells until 20 weeks in PKD peripheral blood, transplanted with different doses of CD45.1 LSK cells after MoAb conditioning with Plx. This analysis provides insights into the extent and durability of the engraftment of donor cells in different hematopoietic lineages and help evaluate the effectiveness of the conditioning treatment in achieving multilineage engraftment in this specific mouse model.

### Example 4: Infusion of wild-type cells in MoAb plus Plerixafor conditioned PKD mice reverts PKD disease parameters.

Next, we wanted to study if the animals engrafted with wild-type cells reverted the anemic phenotype. Due to the high variability of engraftment observed mice, we classified transplanted animals in two groups, with higher than or lower than 10% engraftment of exogenous cells. We conducted a detailed analysis of red blood cell parameters and spleen weight. We observed a significant reduction in all parameters that reach normal levels in the group engrafted with more than 10% exogenous cells (Figure 8A-C). Normal levels were also observed in spleen weight in this group (Figure 8D). We conducted a more extensive analysis of engraftment in the reverted mice (Figure 8E and F). Engraftment was observed in all donor-derived stem cell compartments (Figure 8E) as well as in hematopoietic organs (Figure 8F). All together, these results demonstrate that the use of the combination of MoAb plus Plx proposed here is able to allow engraftment levels that allows a total and stable reversion of the anemic PKD pathology.

### Example 5: Non-genotoxic conditioning allows lower therapeutic engraftment levels than genotoxic ones

Due to the high variability observed between mice, we aimed to determine the minimum percentage of wild type engrafted cells required to achieve therapeutic levels in C57BL/6 PKD mice. To assess this percentage, we focused on studying reticulocyte levels. The correlation between reticulocyte levels and engraftment demonstrated that a 12.5% donor engraftment was sufficient to recover normal levels of reticulocytes (Figure 9 - red line). Moreover, we compared this therapeutic threshold with the one observed using a conventional irradiation conditioning, observing that a recovery of reticulocyte levels was achievable with less engraftment levels when a non-genotoxic conditioning was applied (12.5%) than when a genotoxic one was applied (32%; Figure 9 - black line).

### MATERIAL AND METHODS

### Experimental mice

To conduct these studies, four mouse strains were used. The treatment proposed was applied into wild-type C57BL/6 mice and the recombinant mouse AcB55. C57BL/6J (B6) were obtained from the Jackson Laboratory (Bar Harbor, ME) and bred at the CIEMAT animal facility (registration number 28079-21 A). The AcB55 recombinant mice (also recorded as PKD mice) carrying the 269T>A loss-of-function mutation in the Pklr gene were obtained from Emerillon Therapeutics (Montreal, Quebec, Canada). As the genetic background of ACB55 was both 87,5 % A/J + 12,5 % C57BL/6J we backcrossed onto an inbred mouse strain C57BL/6J for N7 generations. At the end we obtain an incipient congenic strain with a 99,3 % recipient genome. RFP C57BL/6 mice were used as recipients for secondary transplant experiments and congenic donor mice were CD45.1⁺ (B6.SJL-Ptprca Pepcb/BoyJ) were used as donors for primary transplants. This transgenic mouse was generated in the B6SJL-PtprcaPep3b/BoyJ strain (Pep3B; Ly5.1 phenotype), congenic with C57BL/6J (B6; Ly5.2) mice.

Mice were maintained under standard conditions (high-efficiency particulate [HEPA]-filtered air, regulated temperature of 22 °C, light/dark cycle of 12 hours, and food and ultraviolet- irradiated water *ad libitum*) and routinely screened for pathogens. All experimental procedures were carried out according to Spanish and European regulations. Mice used in the experiment were 6-12 weeks old, and both male and female mice were used. Transplant recipients within each experiment were age-matched and randomized to experimental groups.

### Conditioning treatment

Mice were conditioned with a combination of 6 different antibodies over a period of seven days prior to transplantation. Day 0 correspond to the day of transplantation. For anti-CD47 (clone mIAP410), mice received 100µg on Day -7, followed by 500µg daily from Day -5 through Day -2. For anti-c-kit (clone ACK2), which was injected intravenous 500µg was given on Day -5. Fifteen minutes prior to anti-KIT injections, mice received 400µg of diphenhydramine intraperitoneally. Both anti-CD4 (clone GK1.5) and anti-CD8 (clone YTS169.4) were given as 100µg injections daily from Day -2 through Day 0. For anti-CD122 (clone Tm-β1), 250µg was given on Day -2. For anti-CD40L (clone MR-1), 500µg was given on Day 0. The protocol was modified from George BM et al., 2019. Mobilized mice received one dose of Plerixafor (Mozobil. 5mg/Kg) subcutaneously on Day -5 one hour before anti-c-kit injection.

### Transplantation procedure

Total bone marrow was obtained from femur and tibia of donor mice (CD45.1) at day 0 by flushing with phosphate-buffered saline (PBS) using a 25-gauge needle. Cells were resuspended and stained to sort LSK markers including linage cocktail (CD3, CD11b, Gr1, Ter119 and B220), CD117 (c-kit), Sca-1. Sorting was performed in an Influx^{™} (BD) and between 15.000 to 100.000 LSKs cells were transplanted via intravenous injection into conditioned CD45.2 mice or PKD mice. For secondary transplantation, total bone marrow cells were collected at 6 months following the primary transplantation and a range of 5×10⁶-10×10⁶whole bone marrow cells were transplanted via intravenous injection into lethally irradiated RFP mice with two doses of 4.50 Gy spaced 24 h.

### Peripheral blood collection and analysis

Peripheral blood samples were collected every two weeks during the first 3 months after transplantation, followed by monthly collections until the end of the experiment (6 months). Total engraftment and lineage-specific engraftment were analysed by flow cytometry. Additionally, haematological counts were performed using a Sysmex^{™} analyser (XN-Vet1000N) to monitor the evolution of blood parameters over time.

### Spleen, bone marrow and thymus population analysis.

Spleens and thymuses were harvested from conditioned mice and directly mashed through a 40-micrometer filter. For the collection of the BM cells, both tibiae and femora were surgically extracted. BM was harvested by flushing the bones under sterile conditions with PBS (PBS; Sigma-Aldrich, St Louis, MO, USA) + BSA 0.2% buffer and washed once with phosphate buffered saline. The obtained cells from spleens, thymuses, and bone marrow were lysed if required and stained with the following antibodies for engraftment analysis CD45.2-APC (104. Biolegend), CD45.1-PE or CD45.1-BV711 (A20. Biolegend), CD11b-A647 or CD11b-BV711 (M1/70. Biolegend), Gr1-FITC (RB6-8C5. Biolegend), B220-PeFIRE700 (RA3-6B2. Biolegend) or B220-PeCy5 (RA3-6B2. BD Pharmingen) and CD3-APCCy7 (17A2. Biolegend). For erythroid lineage studies, the following antibodies were used Ter119-Alexa 700 (Ter119. Biolegend), CD45.1-PE (A20. Biolegend), CD71-FITC (C2. BD Pharmingen) and CD45.1-BV711 (A20. Biolegend). Last, for long-term HSC chimerism studies BM was stained with CD45.1-PE or CD45.1 PercPCy5.5 (A20. Biolegend), lineage cocktail in FITC including CD3 (145-2C11. BD Pharmingen), Gr1 (RB6-8C5. Biolegend), CD11b (M1/70. eBioscience), Ter119 (Ter119. Biolegend), and stem cell specific markers such as c-kit-BV711 (2B9. Biolegend), Sca1-APCCy7 (D7. Biolegend), CD48-Pecy7 (HM48-1. eBiosciences) and CD150-APC (TC15-12F12.2. Biolegend). Prior to analysis, DAPI or IP was added as a viability stain depending on the antibody panel. Samples were analysed using a BD Fortessa^{™} flow cytometer.

### Statistical analysis

Statistical analysis was conducted using GraphPad Prism v9.4.1. To compare two groups, either Student's t-test or Mann-Whitney U test was utilized based on the normality of data. For the comparison of more than two groups, one-way ANOVA with Tukey's multiple comparison tests was used for normally distributed data, while Kruskal-Wallis test with Dunn's multiple comparison test was employed for nonparametric analyses. Results are presented as mean ± sample SD.

## Claims

1. A composition comprising at least one monoclonal antibody directed against a protein expressed on hematopoietic stem cells for use in a method of treatment of hereditary anemias, bone marrow failures, immunodeficiencies and metabolic diseases in a subject in need thereof, wherein the method of treatment comprises at least one conditioning step, wherein the conditioning step comprises the administration of the composition comprising at least one monoclonal antibody directed against a protein expressed on hematopoietic stem cells and the simultaneous or subsequent administration of at least one hematopoietic stem cell mobilization agent to the subject.

2. A composition comprising at least one monoclonal antibody directed against a protein expressed on hematopoietic stem cells for use in a method of providing stem cell engraftment in a subject, the method comprising at least one conditioning step that comprises simultaneously or subsequently administering to said subject
(i) the composition comprising at least one monoclonal antibody directed against a protein expressed on hematopoietic stem cells, and
(ii) at least one hematopoietic stem cell mobilization agent.

3. The composition for use according to claims 1 or 2, wherein the composition comprising at least one monoclonal antibody directed against a protein expressed on hematopoietic stem cells and the hematopoietic stem cell mobilization agent is administered to the subject prior to the subject receiving an allogeneic or autologous hematopoietic stem cell transplant.

4. The composition for use according to claim 3, wherein the composition comprising at least one monoclonal antibody directed against a protein expressed on hematopoietic cells and the hematopoietic stem cell mobilization agent is administered in a single dose prior to transplant.

5. The composition for use according to any one of the preceding claims, wherein the at least one monoclonal antibody directed against a protein expressed on hematopoietic cells is selected from an anti-CD47 antibody, an anti c-kit (anti-CD117) antibody, an anti-CD45 antibody, an anti-CD135, an anti-CD34, an anti-CD4, an anti-CD3 and an anti-CD38 antibody, or combinations thereof.

6. The composition for use according to any one of the preceding claims, wherein the at least one monoclonal antibody is directed against CD47 and/or c-kit (CD117).

7. The composition for use according to of any one of the preceding claims, wherein the hematopoietic stem cell mobilization agent is selected from the group consisting of Plerixafor, G-CSF, GM-CSF, GM-CSF, BIO5192, or combinations thereof.

8. The composition for use according to of any one of the preceding claims, wherein the hematopoietic stem cell mobilization agent is Plerixafor.

9. The composition for use according to any one of the preceding claims, wherein the administration of the at least one monoclonal antibody directed against a protein expressed on hematopoietic cells and the at least one hematopoietic stem cell mobilization agent is done subsequently, preferably from about 5 min to 3h, from about 20 min to 2 min, from about 40 min to 1.5h, from about 50 min to 1h between first and second administration.

10. The composition for the use of any one of the preceding claims, wherein the conditioning step further comprises administering at least one agent that induces transient immunosuppression in said subject.

11. The composition for the use of claim 10, wherein said agent that induces transient immunosuppression in said subject is selected from the group consisting of agents that inhibit CD4, CD8, CD122 and CD40L, or a combination thereof.

12. The composition for the use of claim 11, wherein said agents that inhibit CD4, CD8, CD122 and CD40L are selected from an anti-CD4 antibody, an anti-CD8 antibody, an anti-CD122 antibody, an anti-CD40L antibody, mycophenolic acid, cyclosporine A, rapamycin, FK506 and corticosteroids, or combinations thereof.

13. The composition for use according to any one of claims 1 and 3 to 12, wherein
a. the hereditary anemias are selected from B-thalassemia, sickle cell disease (SCD) or pyruvate kinase deficiency (PKD), preferably PKD;
b. the bone marrow failures are selected from Fanconi anemia (FA), Blackfan-Diamond anemia (BDA), dyskeratosis congenita (DC), Shwachman-Diamond syndrome (SDS), congenital amegakaryocytic thrombocytopenia (CAMT) or reticular dysgenesis (RD);
c. the immunodeficiencies are selected from adenosine deaminase (ADA) deficiency, X-linked severe combined immunodeficiency (SCID), Wiskott Aldrich or X-linked chronic granulomatous disease (X-CGD);
d. the metabolic diseases are selected from metachromatic leukodystrophy or Mucopolysaccharidosis 1 or 3.
